# EUROPÄISCHE PATENTSCHRIFT

(11) **EP 3 407 732 B1**
(45) Veröffentlichungstag und Bekanntmachung des Hinweises auf die Patenterteilung: **17.06.2020**
(21) Anmeldenummer: 17701706.8
(22) Anmeldetag: 27.01.2017
(51) Int. Cl.: A23K 20/163, A23K 10/30, A23K 20/10, A23K 20/111, A23K 50/30, A23K 50/75, A61K 31/70, C11C 1/00, C11B 11/00

(54) **TIERFUTTERZUSATZ ENTHALTEND DIURNOSID UND/ODER CESTRUMOSID**
ANIMAL FEED ADDITIVE COMPRISING DIURNOSID AND/OR CESTRUMOSIDE
ADDITIF DU FOURRAGE COMPRENANT DIURNOSIDE ET/OU CESTRUMOSIDE

(30) Priorität: 29.01.2016 AT 500492016
(43) Veröffentlichungstag der Anmeldung: 05.12.2018
(73) Patentinhaber: Neufeld GmbH, 2532 Heiligenkreuz (AT)
(72) Erfinder: NEUFELD, Nina, 2532 Heiligenkreuz (AT); NEUFELD, Klaus, 2532 Heiligenkreuz (AT)
(74) Vertreter: Puchberger & Partner Patentanwälte
(86) Internationale Anmeldenummer: PCT/EP2017/051744
(87) Internationale Veröffentlichungsnummer: WO 2017/129732

(56) Entgegenhaltungen:
- EP-A1- 2 070 523
- WO-A1-2007/066355
- WO-A1-2011/127496
- V U AHMAD ET AL.: "A Titogenin Pentasaccharide from Cestrum Diurnum", PHYTOCHEMISTRY, Bd. 34, Nr. 2, 1993, Seiten 511-515, XP002768548,

## Beschreibung

Die Erfindung betrifft einen Tierfutterzusatz zur Anwendung in einem Verfahren für die Proteinkinase-C-Inhibierung.

Osteochondrose/Dyschondroplasie und Beinschwäche sind häufige Gelenkserkrankungen, die hohe wirtschaftliche Verluste bei Broilern, Legehennen, Puten, Mastschweinen, Zuchtsauen, Wiederkäuern, aber auch hohe Therapiekosten bei Pferden und Hunden verursachen. Die genaue Ursache der Osteochondrose/Dyschondroplasie und der Beinschwäche konnte bisher nicht klar definiert werden, die Konsequenz ist jedoch in den häufigsten Fällen bei allen Tierarten eine Knochendeformation mit folgender Lahmheit. Diese Problemkreise treten insbesondere bei jenen Tieren auf, die über ein sehr rasches Körperwachstum verfügen.

In der Praxis werden die Osteochondrose/Dyschondroplasie und die Beinschwäche mit mäßigem Erfolg mit sogenannten nichtsteroidalen Antiphlogistika behandelt. Das ist beispielsweise Acetylsalicylsäure, welche in großen Mengen vor allem beim Geflügel und Schwein eingesetzt wird. Für Legehennen und laktierende Rinder darf dieser Wirkstoff jedoch nicht verwendet werden. Bei anderen landwirtschaftlichen Nutztierarten gibt es für nichtsteroidale Antiphlogistika sogenannte Absetzfristen, das heißt der Wirkstoff darf einige Zeit vor der Schlachtung der Tiere nicht appliziert werden. Erfahrungsgemäß hat sich jedoch gezeigt, dass die Probleme mit Osteochondrose/Dyschondroplasie und mit Beinschwäche insbesondere am Ende der Mastperiode, wenn die Tiere schon ein hohes Körpergewicht erlangt haben, auftreten. Dieser Zeitraum überschneidet sich häufig mit dem Zeitraum der Absetzfrist. Grundsätzlich sind die Landwirtschaft und der Gesetzgeber bestrebt, den Einsatz von Arzneimitteln bei landwirtschaftlichen Nutztieren zu reduzieren. Aus wirtschaftlicher Sicht besteht daher Bedarf für Wirkstoffe, die vorbeugend die Entstehung der Osteochondrose/Dyschondroplasie und der Beinschwäche verhindern können, da zum Zeitpunkt des Eintretens der Veränderungen bereits erhebliche wirtschaftliche Verluste auftreten.

Urolithiasis ist ein insbesondere bei Geflügel häufig auftretendes pathologisches Geschehen, bei dem es zur Ablagerung von Mineralstoffkomplexen im Bereich des Urogenitaltraktes kommt. Die Urolithiasis führt zu einer Obstruktion der ableitenden Harnwege und in den meisten Fällen in der Folge zu einer Funktionsstörung der Nieren. Die ökonomische Bedeutung der Urolithiasis ist insbesondere bei Legehennen erheblich, da sie einerseits zu einer verringerten Legeleistung andererseits auch zu einer erhöhten Mortalitätsrate führen kann.

Überraschenderweise hat sich gezeigt, dass die Verabreichung eines Tierfutterzusatzes/Tierfutters, worin ein pflanzlicher Inhibitor der Proteinkinase C (PKc-Inhibitor), ausgewählt aus Diurnosid, Cestrumosid und deren Mischungen, enthalten ist, zu einem deutlich reduzierten Auftreten von Osteochondrose/Dyschondroplasie und Beinschwäche (leg weakness) führt. Überraschenderweise hat sich auch gezeigt, dass die Verabreichung eines solchen Tierfutterzusatzes/Tierfutters zu einer deutlichen Reduktion der Prävalenz der Urolithiasis insbesondere bei Nutzgeflügel führt.

Die Erfindung betrifft daher in einem Aspekt einen Tierfutterzusatz zur Verwendung zur Prophylaxe von Dyschondroplasie, Osteochondrose und/oder Beinschwäche oder zur Prophylaxe von Urolithiasis bei Nutztieren, Heimtieren und Hobbytieren, wobei der Tierfutterzusatz einen aus Diurnosid, Cestrumosid und deren Mischungen ausgewählten Proteinkinase-C-Inhibitor enthält.

Saccharide aus Cestrum diurnum sind beispielsweise aus dem Artikel "A Titogenin Pentasaccharide from Cestrum Diurnum" von V.U. Ahmad et al., erschienen in Phytochemistry, Vol. 34, Nr. 2, S. 511-515, 1993 bekannt. In diesem Artikel wird die Isolierung von Diurnosid aus Pflanzenmaterial beschrieben. Die WO 2007/066355 A1 beschreibt die Herstellung von angereicherten Extrakten aus Blättern und Blüten von Cestrum diurnum und deren Verwendung als Tierfutterzusatz.

Aufgrund der Tatsache, dass die Wirkstoffe Diurnosid und Cestrumosid kostspielig sind, wurde nach Substanzen gesucht, die einen ökonomischen Einsatz von Diurnosid und Cestrumosid ermöglichen. Im Rahmen von verschiedenen Versuchsreihen wurden unterschiedliche Substanzen ausgetestet.

In überraschender Weise wurde gefunden, dass die einzusetzende Menge Diurnosid und/oder Cestrumosid bei gleicher Wirkung, beispielsweise Bioverfügbarkeit, herabgesetzt werden kann, wenn diese Wirkstoffe in Kombination mit einem oder mehreren optionalen Inhaltsstoffen, ausgewählt aus Magnolol, Honokiol und deren Mischungen, Pilzmyzel von Aspergillus niger, Pilzmycel von Anspergillus oryzae und Chelerythrin, eingesetzt werden. Mit Hilfe der genannten optionalen Inhaltsstoffe ist es gelungen, die wirksame Dosierung von Cestrumosid und Diurnosid um mehr als die Hälfte zu reduzieren. Diese Vorgangsweise gestattet einen ökonomischeren Einsatz in der Praxis.

Die WO 2011/127496 A1 beschreibt einen Tierfutterzusatz, welcher einen alkaloidhältigen Pflanzenextrakt und einen Extrakt bzw. Material aus der Gattung Magnolia enthält. Die EP 2 070 523 A1 beschreibt eine Zusammensetzung mit Magnolol und Honokiol in synergistischer Menge, sowie die Verwendung dieser Mischung als Medikament. Keine der genannten Druckschriften beschreibt die Kombination mit Cestrumosid und/oder Diurnosid für die erfindungsgemäße Anwendung.

Die Erfindung betrifft daher in einer Ausführungsform einen Tierfutterzusatz zur Anwendung in einem Verfahren für die Proteinkinase-C-Inhibierung, der den aus Diurnosid, Cestrumosid und deren Mischungen ausgewählten Wirkstoff in Kombination mit einem oder mehreren weiteren Inhaltsstoffen, ausgewählt aus Magnolol, Honokiol und deren Mischungen, Pilzmyzel von Aspergillus niger, Pilzmyzel von Aspergillus oryzae und Chelerythrin, enthält.

In einer Ausführungsform der Erfindung enthält der Tierfutterzusatz den aus Diurnosid, Cestrumosid und deren Mischungen ausgewählten Wirkstoff in Kombination mit Magnolol und/oder Honokiol.

In einer weiteren Ausführungsform enthält der Tierfutterzusatz den aus Diurnosid, Cestrumosid und deren Mischungen ausgewählten Wirkstoff in Kombination mit Pilzmyzel von Aspergillus niger oder Aspergillus oryzae.

In einer weiteren Ausführungsform enthält der Tierfutterzusatz den aus Diurnosid, Cestrumosid und deren Mischungen ausgewählten Wirkstoff in Kombination mit Chelerythrin.

Der in dem erfindungsgemäßen Tierfutterzusatz enthaltene PKc-Inhibitor sowie optional enthaltene Inhaltsstoffe können natürlichen oder synthetischen Ursprungs sein.
Ein PKc-Inhibitor oder optional enthaltener Inhaltsstoff natürlichen Ursprungs kann beispielsweise in Form von Pflanzenmaterialien und/oder Pflanzenextrakten vorliegen. Die Herstellung der Pflanzenextrakte kann nach an sich bekannten Verfahren erfolgen, wie Fest-flüssig-Extraktion mit Wasser, Heißwasser oder Dampf oder mit organischen Lösungsmitteln wie etwa Ethanol oder Methanol oder mit Lösungsmittelgemischen oder Gemischen solcher Lösungsmittel mit Wasser oder mittels Extraktion mit superkritischem CO₂ als Extraktionsmittel. Bevorzugt wird die Extraktion bei Temperaturen im Bereich von 20 °C bis 160 °C durchgeführt. Die Extrakte werden durch Vermischen einzelner Chargen oder durch Vermischen mit inerten Trägersubstanzen auf den gewünschten Wirkstoffgehalt eingestellt. Die Analytik der Wirkstoffgehalte erfolgt mittels HPLC.

Gemäß einer Ausführungsform enthält der Tierfutterzusatz den aus Diurnosid, Cestrumosid und deren Mischungen ausgewählten PKc-Inhibitor in Kombination mit einem Vitamin-D3-Metaboliten, vorzugsweise ausgewählt aus Calcifediol und Calcitriol. Vorzugsweise liegt das Verhältnis zwischen PKc-Inhibitor und Vitamin-D3-Metaboliten im Bereich von 1:1000 bis 1000:1, bevorzugter im Bereich von 1:100 bis 100:1.

Der erfindungsgemäße Tierfutterzusatz umfasst den aus Diurnosid und/oder Cestrumosid ausgewählten PKc-Inhibitor, der optional in Kombination mit einem oder mehreren weiteren Inhaltsstoffen, ausgewählt aus Magnolol, Honokiol und deren Mischungen, Pilzmyzel von Aspergillus niger, Pilzmyzel von Aspergillus oryzae und Chelerythrin, vorliegt, zusammen mit einem Trägerstoff zur einfacheren Dosierung des Wirkstoffes. Dieser Trägerstoff kann beispielsweise aus pflanzlichen oder mineralischen Trägerstoffen ausgewählt sein, wie etwa Futtermehl, Nachmehl, pflanzliche Grünmehle, Kleien, Tonminerale, Zeolithe, Silikate etc.

Der aus Diurnosid, Cestrumosid und deren Mischungen ausgewählte PKc-Inbibitor liegt in dem erfindungsgemäßen Tierfutterzusatz bevorzugt in einer Dosierung von 0,1 mg bis 20 000 mg, bevorzugter 1 mg bis 1000 mg, pro kg Tierfutterzusatz vor.

Die Dosierung von Magnolol und/oder Honokiol in dem erfindungsgemäßen Tierfutterzusatz liegt bevorzugt im Bereich von 0,5 g bis 50 g pro kg Tierfutterzusatz.

Die Dosierung von Pilzmyzel von Aspergillus niger oder Aspergillus oryzae in dem erfindungsgemäßen Tierfutterzusatz liegt bevorzugt im Bereich von 500 000 mg bis 999 999,9 mg/kg Tierfutterzusatz.

Die Dosierung von Chelerythrin in dem erfindungsgemäßen Tierfutterzusatz liegt bevorzugt im Bereich von 0,1 g bis 10 g pro kg Tierfutterzusatz.

Die Erfindung betrifft auch ein Tierfutter, das einen Tierfutterzusatz, wie oben beschrieben, zur Verwendung zur Prophylaxe von Dyschondroplasie, Osteochondrose und/oder Beinschwäche oder zur Prophylaxe von Urolithiasis bei Nutztieren, Heimtieren und Hobbytieren zusammen mit mindestens einer Futtermittelkomponente, ausgewählt aus der Gruppe bestehend aus Proteinträgern, Kohlenhydratträgern, Raufutter, Silagen, Fetten, Vitaminen, Mineralstoffen und Spurenelementen, umfasst.

Das erfindungsgemäße Tierfutter enthält den aus Diurnosid, Cestrumosid und deren Mischungen ausgewählten PKc-Inhibitor vorzugsweise in einer Dosierung von 0,0001 mg bis 3 mg pro Kilogramm Futter, bezogen auf Futter mit 88% Trockensubstanz. Höhere Dosierungen sind möglich und wirksam, verteuern aber den Einsatz im Futtermittel.

In weiteren Ausführungsformen enthält das erfindungsgemäße Tierfutter den aus Diurnosid, Cestrumosid und deren Mischungen ausgewählten PKc-Inhibitor in Kombination mit einem oder mehreren optionalen Inhaltsstoffen, ausgewählt aus Magnonol, Honokiol und deren Mischungen, Pilzmyzel von Aspergillus niger, Pilzmycel von Aspergillus oryzae und Chelerythrin.

Die Dosierung von Magnolol und/oder Honokiol in dem Tierfutter liegt bevorzugt im Bereich von 0,1 mg bis 10 mg pro kg Futtermittel, bevorzugter im Bereich von 0,3 mg bis 3 mg pro kg Futtermittel (bezogen auf Futter mit 88% Trockensubstanz).

Die Dosierung von Pilzmyzel von Aspergillus niger oder Aspergillus oryzae in dem Tierfutter liegt bevorzugt im Bereich von 50 mg bis 5000 mg pro kg Futtermittel, bevorzugter im Bereich von 200 bis 2000 mg pro kg Futtermittel (bezogen auf Futter mit 88% Trockensubstanz).

Die Dosierung von Chelerythrin in dem Tierfutter liegt bevorzugt im Bereich von 0,05 mg bis 5 mg pro kg Futtermittel (bezogen auf Futter mit 88% Trockensubstanz).

Die Erfindung betrifft auch eine Vormischung zur Herstellung eines Tierfutters. Die Vormischung enthält einen Tierfutterzusatz gemäß der Erfindung zusammen mit mindestens einer Futtermittelkomponente, ausgewählt aus der Gruppe bestehend aus Proteinträgern, Kohlenhydratträgern, Raufutter, Silagen, Fetten, Vitaminen, Mineralstoffen und Spurenelementen.

Gemäß einem weiteren Merkmal betrifft die Erfindung die Verwendung eines aus Diurnosid und/oder Cestrumosid ausgewählten Proteinkinase-C-Inhibitors in einem Tierfutterzusatz oder Tränkwasserzusatz zur Prophylaxe von Dyschondroplasie, Osteochondrose und/oder Beinschwäche oder zur Prophylaxe von Urolithiasis bei Nutztieren, Heimtieren und Hobbytieren. Der Proteinkinase-C-Inhibitor wird dem Tier bevorzugt in einer Menge von von 0,00001 mg bis 0,3 mg pro Kilogramm Körpermasse pro Tag verabreicht.

Die Erfindung betrifft auch die Verwendung eines aus Diurnosid und/oder Cestrumosid ausgewählten Proteinkinase-C-Inhibitors in Kombination mit einem oder mehreren optionalen Inhaltsstoffen, ausgewählt aus Magnolol, Honokiol und deren Mischungen, Pilzmyzel von Aspergillus niger oder Aspergillus oryzae und Chelerythrin, in einem Tierfutterzusatz oder Tränkwasserzusatz zur Prophylaxe von Dyschondroplasie, Osteochondrose und/oder Beinschwäche oder zur Prophylaxe von Urolithiasis bei Nutztieren, Heimtieren und Hobbytieren.

Magnolol und/oder Honokiol wird dem Tier bevorzugt in einer Menge von 0,004 mg bis 1 mg pro Kilogramm Körpermasse pro Tag verabreicht.

Pilzmyzel von Aspergillus niger oder Aspergillus oryzae wird dem Tier bevorzugt in einer Menge von 2 mg bis 500 mg pro Kilogramm Körpermasse pro Tag verabreicht.

Chelerythrin wird dem Tier bevorzugt in einer Menge von 0,002 mg bis 0,5 mg pro Kilogramm Körpermasse pro Tag verabreicht.

Der erfindungsgemäße Tierfutterzusatz kann auch ein Tränkwasserzusatz sein. Die zweckmäßigen Verabreichungsmengen der Wirkstoffe und optionalen Inhaltsstoffe können den vorstehend angeführten Mengen pro kg Körpermasse pro Tag entsprechen.

Die Erfindung betrifft auch die Verwendung eines aus Diurnosid und/oder Cestrumosid ausgewählten Proteinkinase-C-Inhibitors, gegebenenfalls in Kombination mit einem oder mehreren optionalen Inhaltsstoffen, ausgewählt aus Magnolol, Honokiol und deren Mischungen, Pilzmyzel von Aspergillus niger oder Aspergillus oryzae und Chelerythrin, zur Herstellung eines Tierfutterzusatzes oder eines Tierfutters oder einer Tierfutter-Vormischung oder eines Tränkwasserzusatzes zur Verwendung zur Prophylaxe von Dyschondroplasie, Osteochondrose und/oder Beinschwäche oder zur Prophylaxe von Urolithiasis bei Nutztieren, Heimtieren und Hobbytieren.

Im Folgenden wird die Erfindung anhand von Beispielen unter Bezugnahme auf die Figuren näher erläutert, wobei
Fig. 1 das Auftreten von Osteochondrose in Fütterungsversuchen mit Broilern unter Verwendung erfindungsgemäßer Tierfutterzusätze im Vergleich zur Nullkontrollgruppe, einer Kontrollgruppe mit Calcitriol und einer Kontrollgruppe mit Calcifediol zeigt;
Fig. 2 das Auftreten von Osteochondrose in Fütterungsversuchen mit Broilern unter Verwendung erfindungsgemäßer Tierfutterzusätze im Vergleich zur Kontrollgruppe zeigt;
Fig. 3 das Auftreten von Urolithiasis in Fütterungsversuchen mit Broilern unter Verwendung erfindungsgemäßer Tierfutterzusätze im Vergleich zur Kontrollgruppe zeigt;
Fig. 4 das Auftreten von Osteochondrose in Fütterungsversuchen mit Broilern unter Verwendung erfindungsgemäßer Tierfutterzusätze im Vergleich zur Kontrollgruppe zeigt;
Fig. 5 das Auftreten von Urolithiasis in Fütterungsversuchen mit Broilern unter Verwendung erfindungsgemäßer Tierfutterzusätze im Vergleich zur Kontrollgruppe zeigt;
Fig. 6 das Auftreten von Osteochondrose in Fütterungsversuchen mit Broilern unter Verwendung erfindungsgemäßer Tierfutterzusätze im Vergleich zur Kontrollgruppe zeigt;
Fig. 7 das Auftreten von Urolithiasis in Fütterungsversuchen mit Legehennen unter Verwendung erfindungsgemäßer Tierfutterzusätze im Vergleich zur Kontrollgruppe zeigt;
Fig. 8 das Auftreten von Brustblasen als Indikator für Beinschwäche in Fütterungsversuchen mit Legehennen unter Verwendung erfindungsgemäßer Tierfutterzusätze im Vergleich zur Kontrollgruppe zeigt;
Fig. 9 das Auftreten von Osteochondrose in Fütterungsversuchen mit Mastschweinen unter Verwendung erfindungsgemäßer Tierfutterzusätze im Vergleich zur Kontrollgruppe zeigt;
Fig. 10 das Auftreten von Osteochondrose in Fütterungsversuchen mit Mastschweinen unter Verwendung erfindungsgemäßer Tierfutterzusätze im Vergleich zur Kontrollgruppe zeigt.

### BEISPIELE

### Beispiel 1

In einem kommerziellen Broilermastbetrieb wurden fünf Stallungen mit jeweils rund 43 000 Tieren bezüglich des Auftretens von Osteochondrose/Dyschondroplasie im Feldversuch verglichen. Alle Stallungen wurden mit marktüblichem Broilermastfutter, vertrieben unter der Bezeichnung GeflügelmastKorn® (Firma Garant Tiernahrung), gefüttert, wobei in der Stallung K1 (Kontrolle) keine weiteren Zusatzstoffe zum Einsatz kamen.

Für die Stallung K2 (Kontrollgruppe Calcitriol) wurde in das Broilermastfutter ein Futterzusatz mit einem Gehalt von 20 ppm Calcitriol in einer Menge von 150 ppm (bezogen auf Futter mit 88% Trockensubstanz) eingemischt.

Für die Stallung K2 (Kontrollgruppe Calcifediol) wurde in das Broilermastfutter ein Futterzusatz mit einem Gehalt von 200 ppm Calcifediol in einer Menge von 150 ppm (bezogen auf Futter mit 88% Trockensubstanz) eingemischt.

Für die Stallung V1 (Versuchsgruppe Diurnosid plus Calcitriol) wurde in das Broilermastfutter der erfindungsgemäße Futterzusatz mit einem Gehalt von 100 ppm Diurnosid und 10 ppm Calcitriol in einer Menge von 150 ppm (bezogen auf Futter mit 88% Trockensubstanz) eingemischt.

Für die Stallung V2 (Versuchsgruppe Cestrumosid plus Calcifediol) wurde in das Broilermastfutter der erfindungsgemäße Futterzusatz mit einem Gehalt von 20 ppm Cestrumosid und 100 ppm Calcifediol in einer Menge von 150 ppm (bezogen auf Futter mit 88% Trockensubstanz) eingemischt.

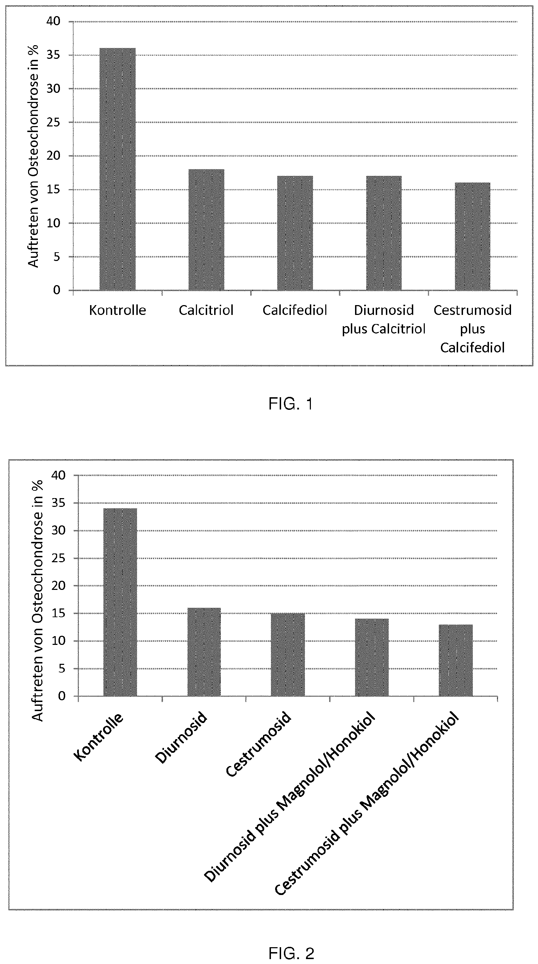

Versuchsbeginn war mit Einstallen der Tiere am 1. Lebenstag und endete mit der Schlachtung der Tiere am 30. Lebenstag. Nach der Schlachtung wurden aus jedem Stall 100 Schlachtkörper willkürlich entnommen. An diesen 500 Schlachtkörpern wurden pathologische Untersuchungen auf Osteochondrose durchgeführt. Die Ergebnisse sind in der Fig. 1 dargestellt. Aus der Fig. 1 ist ersichtlich, dass der erfindungsgemäße Tierfutterzusatz die Inzidenz von Osteochondrose/Dyschondroplasie in einem Broilermastbetrieb unter üblichen Produktionsbedingungen signifikant reduzieren konnte. Weiters ist ersichtlich, dass die wirksamen Einsatzdosierungen für Calcitriol und Calcifediol um die Hälfte reduziert werden konnten. Es konnte im betreffenden Betrieb festgestellt werden, dass die Mobilität der Tiere unter Verwendung der Versuchssubstanzen deutlich besser war als in der Nullkontrollgruppe.

### Beispiel 2

In einem kommerziellen Broilermastbetrieb wurden fünf Stallungen mit jeweils rund 43 000 Tieren bezüglich des Auftretens von Osteochondrose/Dyschondroplasie und Urolithiasis im Feldversuch verglichen. Alle Stallungen wurden mit marktüblichem Broilermastfutter, vertrieben unter der Bezeichnung GeflügelmastKorn® (Firma Garant Tiernahrung), gefüttert, wobei in der Stallung K (Kontrolle) keine weiteren Zusatzstoffe zum Einsatz kamen.

Für die Stallung V1 (Versuchsgruppe Diurnosid) wurde in das Broilermastfutter der erfindungsgemäße Futterzusatz mit einem Gehalt von 100 ppm Diurnosid in einer Menge von 150 ppm (bezogen auf Futter mit 88% Trockensubstanz) eingemischt.

Für die Stallung V2 (Versuchsgruppe Cestrumosid) wurde in das Broilermastfutter der erfindungsgemäße Futterzusatz mit einem Gehalt von 20 ppm Cestrumosid in einer Menge von 150 ppm (bezogen auf Futter mit 88% Trockensubstanz) eingemischt.

Für die Stallung V3 (Versuchsgruppe Diurnosid plus Magnolol/Honokiol) wurde in das Broilermastfutter der erfindungsgemäße Futterzusatz mit einem Gehalt von 100 ppm Diurnosid und 2 % einer Mischung bestehend aus Magnolol/Honokiol (Magnolol/Honokiol im Verhältnis 2:1) in einer Menge von 50 ppm (bezogen auf Futter mit 88% Trockensubstanz) eingemischt.

Für die Stallung V4 (Versuchsgruppe Cestrumosid plus Magnolol/Honokiol) wurde in das Broilermastfutter der erfindungsgemäße Futterzusatz mit einem Gehalt von 20 ppm Cestrumosid und 2 % einer Mischung bestehend aus Magnolol/Honokiol (Magnolol/Honokiol im Verhältnis 2:1) in einer Menge von 50 ppm (bezogen auf Futter mit 88% Trockensubstanz) eingemischt.

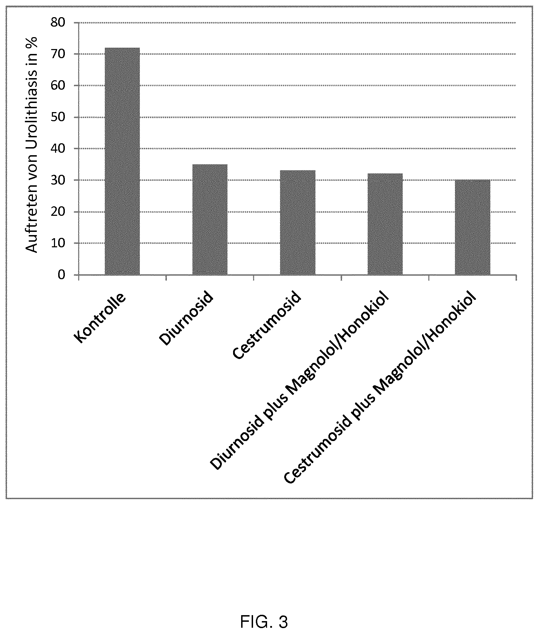

Versuchsbeginn war mit Einstallen der Tiere am 1. Lebenstag und endete mit der Schlachtung der Tiere am 30. Lebenstag. Nach der Schlachtung wurden aus jedem Stall 100 Schlachtkörper willkürlich entnommen. An diesen 500 Schlachtkörpern wurden pathologische Untersuchungen auf Osteochondrose und Urolithiasis (makroskopisch erkennbare Harnsteinbildung) durchgeführt. Die Ergebnisse sind in den Fig. 2 und 3 dargestellt. Aus der Fig. 2 ist ersichtlich, dass der erfindungsgemäße Tierfutterzusatz die Inzidenz von Osteochondrose/Dyschondroplasie in einem Broilermastbetrieb unter üblichen Produktionsbedingungen signifikant reduzieren konnte. Es konnte im betreffenden Betrieb festgestellt werden, dass die Mobilität der Tiere unter Verwendung der Versuchssubstanzen deutlich besser war als in der Kontrollgruppe. Aus der Fig. 3 ist ersichtlich, dass der erfindungsgemäße Tierfutterzusatz die Inzidenz von Urolithiasis in einem Broilermastbetrieb unter üblichen Produktionsbedingungen signifikant reduzieren konnte.

### Beispiel 3

In einem kommerziellen Broilermastbetrieb wurden fünf Stallungen mit jeweils rund 43 000 Tieren bezüglich des Auftretens von Osteochondrose/Dyschondroplasie und Urolithiasis im Feldversuch verglichen. Alle Stallungen wurden mit marktüblichem Broilermastfutter, vertrieben unter der Bezeichnung GeflügelmastKorn® (Firma Garant Tiernahrung), gefüttert, wobei in der Stallung K (Kontrolle) keine weiteren Zusatzstoffe zum Einsatz kamen.

Für die Stallung V1 (Versuchsgruppe Diurnosid plus Aspergillus niger) wurde in das Broilermastfutter der erfindungsgemäße Futterzusatz mit einem Gehalt von 10 ppm Diurnosid und einem Gehalt von 90% Myzel von Aspergillus niger in einer Menge von 500 ppm (bezogen auf Futter mit 88% Trockensubstanz) eingemischt.

Für die Stallung V2 (Versuchsgruppe Cestrumosid plus Aspergillus oryzae) wurde in das Broilermastfutter der erfindungsgemäße Futterzusatz mit einem Gehalt von 2 ppm Cestrumosid und einem Gehalt von 90% Myzel von Aspergillus oryzae in einer Menge von 500 ppm (bezogen auf Futter mit 88% Trockensubstanz) eingemischt.

Für die Stallung V3 (Versuchsgruppe Diurnosid plus Chelerythrin) wurde in das Broilermastfutter der erfindungsgemäße Futterzusatz mit einem Gehalt von 100 ppm Diurnosid und 1% Chelerythrin in einer Menge von 50 ppm (bezogen auf Futter mit 88% Trockensubstanz) eingemischt.

Für die Stallung V4 (Versuchsgruppe Cestrumosid plus Chelerythrin) wurde in das Broilermastfutter der erfindungsgemäße Futterzusatz mit einem Gehalt von 20 ppm Cestrumosid und 1% ppm Chelerythrin in einer Menge von 50 ppm (bezogen auf Futter mit 88% Trockensubstanz) eingemischt.

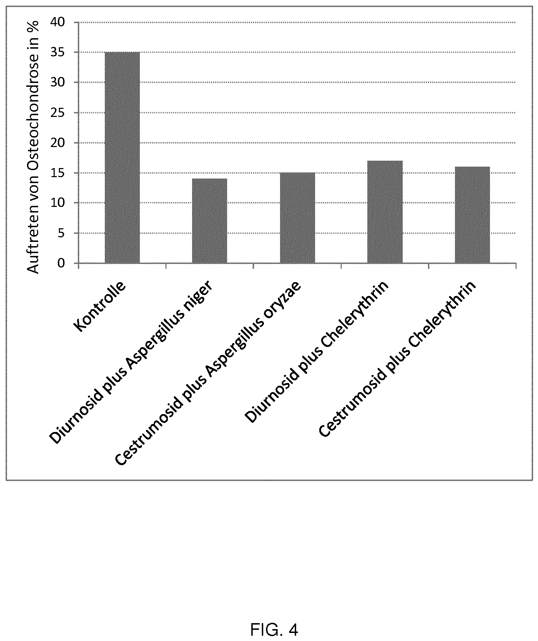

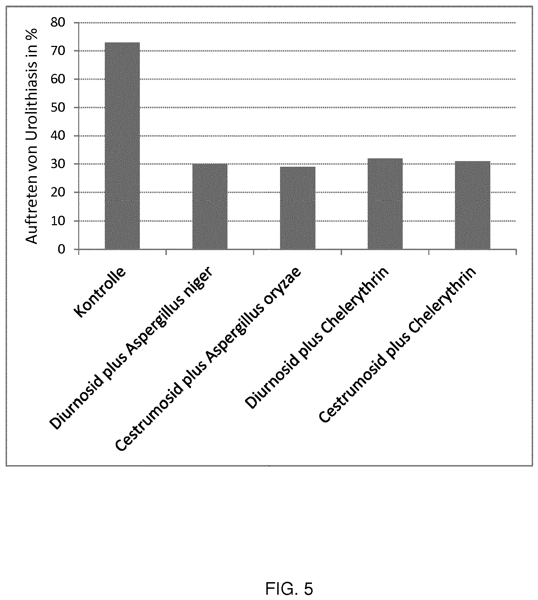

Versuchsbeginn war mit Einstallen der Tiere am 1. Lebenstag und endete mit der Schlachtung der Tiere am 30. Lebenstag. Nach der Schlachtung wurden aus jedem Stall 100 Schlachtkörper willkürlich entnommen. An diesen 500 Schlachtkörpern wurden pathologische Untersuchungen auf Osteochondrose und Urolithiasis durchgeführt. Die Ergebnisse sind in den Fig. 4 und 5 dargestellt. Aus der Fig. 4 ist ersichtlich, dass der erfindungsgemäße Tierfutterzusatz die Inzidenz von Osteochondrose/Dyschondroplasie in einem Broilermastbetrieb unter üblichen Produktionsbedingungen signifikant reduzieren konnte. Es konnte im betreffenden Betrieb festgestellt werden, dass die Mobilität der Tiere unter Verwendung der Versuchssubstanzen deutlich besser war als in der Kontrollgruppe. Aus der Fig. 5 ist ersichtlich, dass der erfindungsgemäße Tierfutterzusatz die Inzidenz von Urolithiasis in einem Broilermastbetrieb unter üblichen Produktionsbedingungen signifikant reduzieren konnte.

### Beispiel 4

In einem kommerziellen Broilermastbetrieb wurden drei Stallungen mit jeweils rund 43 000 Tieren bezüglich des Auftretens von Osteochondrose/Dyschondroplasie und Urolithiasis im Feldversuch verglichen. Alle Stallungen wurden mit marktüblichem Broilermastfutter, vertrieben unter der Bezeichnung GeflügelmastKorn® (Firma Garant Tiernahrung), gefüttert, wobei in der Stallung K (Kontrolle) keine weiteren Zusatzstoffe zum Einsatz kamen.

Für die Stallung V1 (Versuchsgruppe Diurnosid plus Cestrumosid plus Magnolol/Honokiol plus Aspergillus niger) wurde in das Broilermastfutter der erfindungsgemäße Futterzusatz mit einem Gehalt von 10 ppm Diurnosid, 2 ppm Cestrumosid, 1.000 ppm einer Mischung bestehend aus Magnolol/Honokiol (Magnolol/Honokiol im Verhältnis 2:1) und einem Gehalt von 90% Myzel von Aspergillus niger in einer Menge von 500 ppm (bezogen auf Futter mit 88% Trockensubstanz) eingemischt.

Für die Stallung V2 (Versuchsgruppe Diurnosid plus Cestrumosid plus Aspergillus oryzae) wurde in das Broilermastfutter der erfindungsgemäße Futterzusatz mit einem Gehalt von 10 ppm Diurnosid, 2 ppm Cestrumosid und einem Gehalt von 90% Myzel von Aspergillus oryzae in einer Menge von 500 ppm (bezogen auf Futter mit 88% Trockensubstanz) eingemischt.

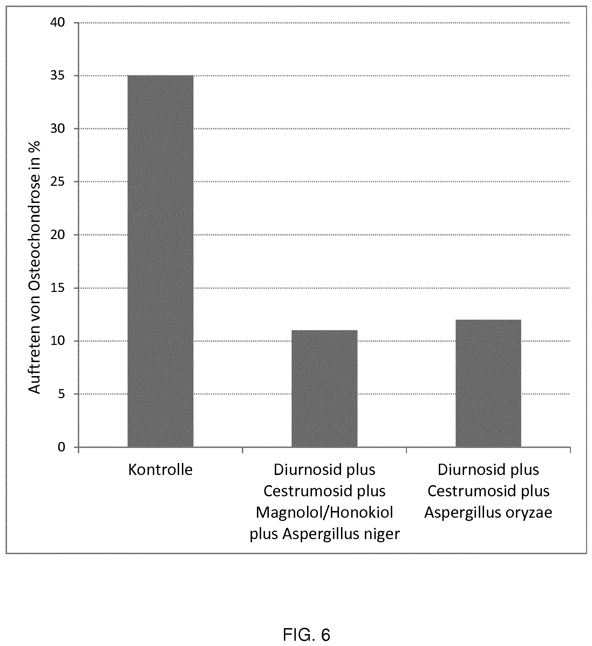

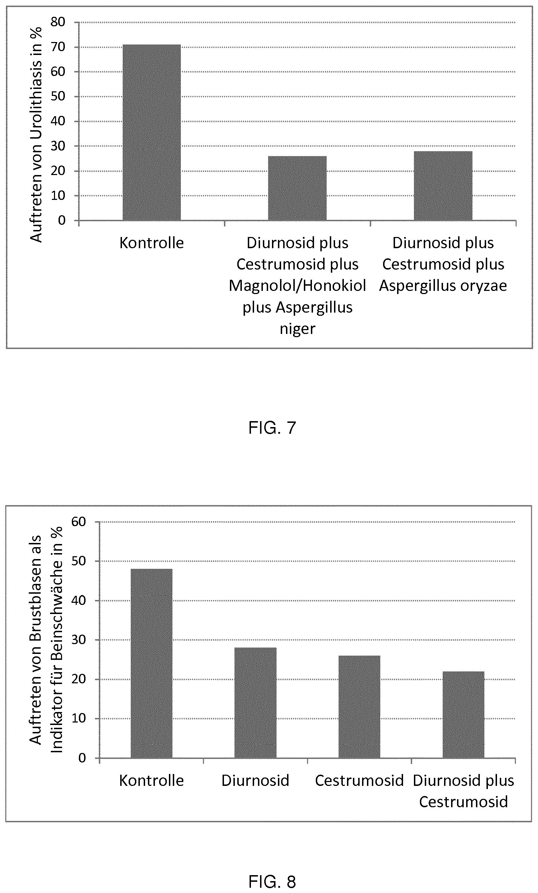

Versuchsbeginn war mit Einstallen der Tiere am 1. Lebenstag und endete mit der Schlachtung der Tiere am 30. Lebenstag. Nach der Schlachtung wurden aus jedem Stall 100 Schlachtkörper willkürlich entnommen. An diesen 300 Schlachtkörpern wurden pathologische Untersuchungen auf Osteochondrose und Urolithiasis durchgeführt. Die Ergebnisse sind in den Fig. 6 und 7 dargestellt. Aus der Fig. 6 ist ersichtlich, dass der erfindungsgemäße Tierfutterzusatz die Inzidenz von Osteochondrose/Dyschondroplasie in einem Broilermastbetrieb unter üblichen Produktionsbedingungen signifikant reduzieren konnte. Es konnte im betreffenden Betrieb festgestellt werden, dass die Mobilität der Tiere unter Verwendung der Versuchssubstanzen deutlich besser war als in der Kontrollgruppe. Aus der Fig. 7 ist ersichtlich, dass der erfindungsgemäße Tierfutterzusatz die Inzidenz von Urolithiasis in einem Broilermastbetrieb unter üblichen Produktionsbedingungen signifikant reduzieren konnte.

### Beispiel 5

In einem Versuchsstall wurden vier Gruppen von Legehennen mit jeweils 50 Tieren pro Gruppe bezüglich des Auftretens von Beinschwäche verglichen. Alle Versuchsgruppen wurden mit marktüblichem Legehennenfutter gefüttert, vertrieben unter der Bezeichnung LegeKorn® (Firma Garant Tiernahrung), wobei im Futter der Kontrollgruppe keine weiteren Zusatzstoffe zum Einsatz kamen.

Für die Versuchsgruppe 1 wurde in das Legealleinfutter ein erfindungsgemäßer Futterzusatz mit einem Gehalt von 100 ppm Diurnosid in einer Menge von 150 ppm (bezogen auf Futter mit 88% Trockensubstanz) eingemischt.

Für die Versuchsgruppe 2 wurde in das Legealleinfutter ein erfindungsgemäßer Futterzusatz mit einem Gehalt von 20 ppm Cestrumosid in einer Menge von 150 ppm (bezogen auf Futter mit 88% Trockensubstanz) eingemischt.

Für die Versuchsgruppe 3 wurde in das Legealleinfutter ein erfindungsgemäßer Futterzusatz mit einem Gehalt von 50 ppm Diurnosid und 10 ppm Cestrumosid in einer Menge von 150 ppm (bezogen auf Futter mit 88% Trockensubstanz) eingemischt.

Versuchsbeginn war in der 19. Lebenswoche der Tiere. Die Tiere wurden in vier Abteilungen in Bodenhaltung auf Stroh als Einstreu gehalten. Die Nester zur Eiablage waren abgetrennt und ebenfalls mit Stroh als Einstreumaterial ausgestattet. Der Versuch endete nach 15 Monaten mit der Schlachtung der Tiere. Nach der Schlachtung wurden alle Schlachtkörper einer makroskopischen pathologischen Untersuchung auf Brustblasenbildung (breast blisters) unterzogen. Als Brustblase wird eine Zyste zwischen Brustbein und der darüber liegenden Haut bezeichnet, die durch vermehrtes Liegen auf dem Brustbereich ausgelöst wird. Da diese Art des Liegens bei Geflügel mit Beinschwäche häufiger auftritt als bei gesunden Tieren, wurde die Häufigkeit von Brustblasen als Indikator für eine eingeschränkte Mobilität und somit für Beinschwäche herangezogen. Die Ergebnisse des Versuchs sind in der Fig. 8 dargestellt. Die Fig. 8 zeigt, dass unter Verwendung der betreffenden Versuchssubstanzen das Auftreten von Brustblasen als Indikator für Beinschwäche signifikant gegenüber der Kontrollgruppe reduziert war.

### Beispiel 6

In einem Versuchsstall wurden fünf Gruppen von männlichen Mastschweinen mit jeweils 20 Tieren pro Gruppe bezüglich des Auftretens von Osteochondrose verglichen. Die Tiere wurden mit einem Durchschnittsgewicht pro Gruppe von 30 kg eingestallt. Alle Versuchsgruppen wurden mit marktüblichem Schweinemastfutter gefüttert, vertrieben unter der Bezeichnung SchweinemastKorn® OGT (Firma Garant Tiernahrung), wobei im Futter der Kontrollgruppe keine weiteren Zusatzstoffe zum Einsatz kamen.

Für die Versuchsgruppe 1 wurde in das Schweinemastfutter ein erfindungsgemäßer Futterzusatz mit einem Gehalt von 100 ppm Diurnosid in einer Menge von 100 ppm (bezogen auf Futter mit 88% Trockensubstanz) eingemischt.

Für die Versuchsgruppe 2 wurde in das Schweinemastfutter ein erfindungsgemäßer Futterzusatz mit einem Gehalt von 20 ppm Cestrumosid in einer Menge von 100 ppm (bezogen auf Futter mit 88% Trockensubstanz) eingemischt.

Für die Versuchsgruppe 3 wurde in das Schweinemastfutter ein erfindungsgemäßer Futterzusatz mit einem Gehalt von 10 ppm Diurnosid und einem Gehalt von 90 % Myzel von Aspergillus oryzae in einer Menge von 500 ppm (bezogen auf Futter mit 88% Trockensubstanz) eingemischt.

Für die Versuchsgruppe 4 wurde in das Schweinemastfutter ein erfindungsgemäßer Futterzusatz mit einem Gehalt von 2 ppm Cestrumosid und einem Gehalt von 90 % Myzel von Aspergillus niger in einer Menge von 500 ppm (bezogen auf Futter mit 88% Trockensubstanz) eingemischt.

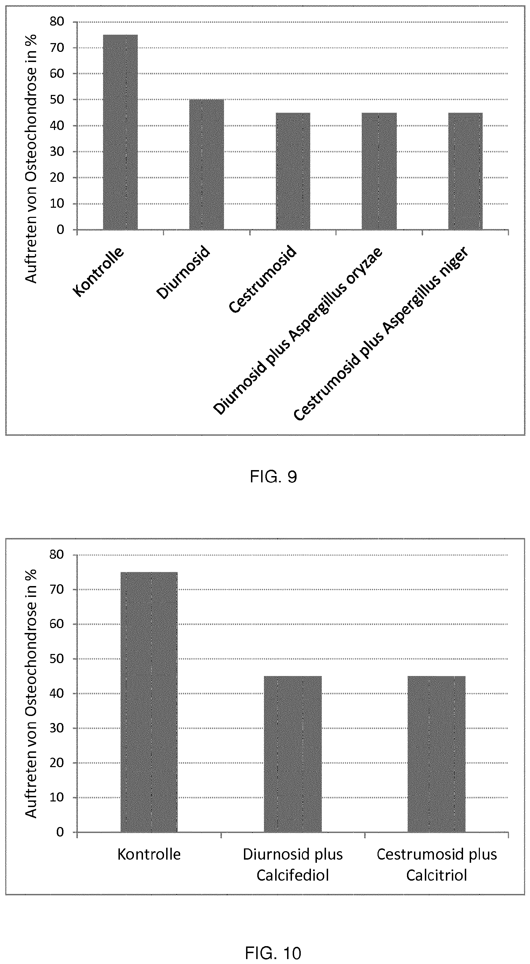

Die Tiere wurden bis zu einem durchschnittlichen Gewicht von 100 kg pro Gruppe gemästet. Der Versuch endete mit der Schlachtung der Tiere. Nach der Schlachtung wurden bei allen Schlachtkörper jeweils das rechte Kniegelenk (Articulatio genus) einer makroskopischen pathologischen Untersuchung auf Osteochondrose unterzogen. Die Ergebnisse des Versuchs sind in der Fig. 9 dargestellt. Die Fig. 9 zeigt, dass die Inzidenz von Osteochondrose am rechten Kniegelenk unter Verwendung der erfindungsgemäßen Substanzen signifikant geringer war als bei der Kontrollgruppe. Es konnten auch in den Versuchsgruppen tendenziell weniger Tiere mit Mobilitätsstörungen (sitzend) beobachtet werden.

### Beispiel 7

In einem Versuchsstall wurden drei Gruppen von männlichen Mastschweinen mit jeweils 20 Tieren pro Gruppe bezüglich des Auftretens von Osteochondrose verglichen. Die Tiere wurden mit einem Durchschnittsgewicht pro Gruppe von 30 kg eingestallt. Alle Versuchsgruppen wurden mit marktüblichem Schweinemastfutter gefüttert, vertrieben unter der Bezeichnung SchweinemastKorn® OGT (Firma Garant Tiernahrung), wobei im Futter der Kontrollgruppe keine weiteren Zusatzstoffe zum Einsatz kamen.

Für die Versuchsgruppe 1 wurde in das Schweinemastfutter ein erfindungsgemäßer Futterzusatz mit einem Gehalt von 100 ppm Diurnosid und 10 ppm Calcifediol in einer Menge von 100 ppm (bezogen auf Futter mit 88% Trockensubstanz) eingemischt.

Für die Versuchsgruppe 2 wurde in das Schweinemastfutter ein erfindungsgemäßer Futterzusatz mit einem Gehalt von 20 ppm Cestrumosid und 10 ppm Calcitriol in einer Menge von 100 ppm (bezogen auf Futter mit 88% Trockensubstanz) eingemischt.

Die Tiere wurden bis zu einem durchschnittlichen Gewicht von 100 kg pro Gruppe gemästet. Der Versuch endete mit der Schlachtung der Tiere. Nach der Schlachtung wurden bei allen Schlachtkörper jeweils das rechte Kniegelenk (Articulatio genus) einer makroskopischen pathologischen Untersuchung auf Osteochondrose unterzogen. Die Ergebnisse des Versuchs sind in der Fig. 10 dargestellt. Die Fig. 10 zeigt, dass der erfindungsgemäße Tierfutterzusatz zu einer signifikanten Reduktion des Auftretens von Osteochondrose bei Mastschweinen führte.

## Patentansprüche

1. Tierfutterzusatz zur Anwendung in einem Verfahren für die Proteinkinase-C-Inhibierung zur Prophylaxe von Dyschondroplasie, Osteochondrose und/oder Beinschwäche oder zur Prophylaxe von Urolithiasis bei Nutztieren, Heimtieren und Hobbytieren, **dadurch gekennzeichnet, dass** der Tierfutterzusatz Diurnosid, Cestrumosid oder deren Mischung in Kombination mit einem oder mehreren weiteren Inhaltsstoffen, ausgewählt aus Magnolol, Honokiol und deren Mischungen, Pilzmyzel von Aspergillus niger, Pilzmyzel von Aspergillus oryzae und Chelerythrin, aufweist.

2. Tierfutterzusatz zur Anwendung nach Anspruch 1, **dadurch gekennzeichnet, dass** er Diurnosid, Cestrumosid oder deren Mischung in Kombination mit Magnolol und/oder Honokiol enthält.

3. Tierfutterzusatz zur Anwendung nach Anspruch 1, **dadurch gekennzeichnet, dass** er Diurnosid. Cestrumosid oder deren Mischung in Kombination mit Pilzmyzel von Aspergillus niger oder Aspergillus oryzae enthält.

4. Tierfutterzusatz zur Anwendung nach Anspruch 1, **dadurch gekennzeichnet, dass** er Diurnosid, Cestrumosid oder deren Mischung in Kombination mit Chelerythrin enthält.

5. Tierfutterzusatz zur Anwendung nach einem der Ansprüche 1 bis 4 zur Verwendung zur Prophylaxe von Dyschondroplasie, Osteochondrose und/oder Beinschwäche oder zur Prophylaxe von Urolithiasis bei Nutztieren, Heimtieren und Hobbytieren.

6. Tierfutterzusatz zur Anwendung nach einem der Ansprüche 1 bis 5, **dadurch gekennzeichnet, dass** der aus Diurnosid, Cestrumosidund deren Mischungen ausgewählte Wirkstoff in Kombination mit einem Vitamin-D3-Metaboliten, vorzugsweise ausgewählt aus Calcifediol und Calcitriol, vorliegt.

7. Tierfutterzusatz zur Anwendung nach Anspruch 6, **dadurch gekennzeichnet, dass** das Verhältnis zwischen dem aus Diurnosid, Cestrumosid und deren Mischungen ausgewählten Wirkstoff und dem vorzugsweise aus Calcifediol und Calcitriol ausgewählten Vitamin-D3-Metaboliten im Bereich von 1:1 000 bis 1 000:1, vorzugsweise im Bereich von 1:100 bis 100:1, liegt.

8. Tierfutterzusatz zur Anwendung nach Anspruch 1, **dadurch gekennzeichnet, dass** der aus Diurnosid, Cestrumosid und deren Mischungen ausgewählte Wirkstoff in einer Dosierung von 0,1 mg bis 20 000 mg pro kg Tierfutterzusatz vorliegt.

9. Tierfutter zur Verwendung zur Prophylaxe von Dyschondroplasie, Osteochondrose und/oder Beinschwäche oder zur Prophylaxe von Urolithiasis bei Nutztieren, Heimtieren und Hobbytieren, **dadurch gekennzeichnet, dass** es einen Tierfutterzusatz zur Anwendung nach einem der Ansprüche 1-8 zusammen mit mindestens einer Futtermittelkomponente, ausgewählt aus der Gruppe bestehend aus Proteinträgern, Kohlenhydratträgern, Raufutter, Silagen, Fetten, Vitaminen, Mineralstoffen und Spurenelementen, enthält.

10. Tierfutter zur Verwendung nach Anspruch 9, **dadurch gekennzeichnet, dass** es den aus Diurnosid, Cestrumosid und deren Mischungen ausgewählten Wirkstoff in einer Dosierung von 0,0001 mg bis 3 mg pro Kilogramm Futter, bezogen auf Futter mit 88%Trockensubstanz, enthält.

11. Vormischung zur Herstellung eines Tierfutters zur Verwendung nach Anspruch 9 oder 10 **dadurch gekennzeichnet, dass** sie einen Tierfutterzusatz zur Anwendung nach einem der Ansprüche 1 bis 8 zusammen mit mindestens einer Futtermittelkomponente, ausgewählt aus der Gruppe bestehend aus Proteinträgern, Kohlenhydratträgern, Raufutter, Silagen, Fetten, Vitaminen, Mineralstoffen und Spurenelementen, enthält.

12. Tierfutterzusatz zur Anwendung nach einem der Ansprüche 1 bis 8 **dadurch gekennzeichnet, dass** der Wirkstoff dem Tier in einer Menge von 0,00001 mg bis 0,3 mg pro Kilogramm Körpergewicht pro Tag verabreicht wird.

## Claims

1. An animal feed additive for use in a process for protein kinase C inhibition for the prophylaxis of dyschondroplasia, osteochondrosis and/or leg weakness or for the prophylaxis of urolithiasis in farm animals, pets and hobby animals, **characterized in that** the animal feed additive comprises diurnoside, cestrumoside or a mixture thereof in combination with one or more further ingredients selected from the group consisting of magnolol, honokiol and mixtures thereof, fungal mycelium of Aspergillus niger, fungal mycelium of Aspergillus oryzae and chelerythrine.

2. The animal feed additive for use according to claim 1, **characterized in that** it contains diurnoside, cestrumoside or a mixture thereof in combination with magnolol and/or honokiol.

3. The animal feed additive for use according to claim 1, **characterized in that** it contains diurnoside, cestrumoside or a mixture thereof in combination with fungal mycelium of Aspergillus niger or Aspergillus oryzae.

4. The animal feed additive for use according to claim 1, **characterized in that** it contains diurnoside, cestrumoside or a mixture thereof in combination with chelerythrine.

5. The animal feed additive for use according to one of claims 1 to 4 for use for the prophylaxis of dyschondroplasia, osteochondrosis and/or leg weakness or for the prophylaxis of urolithiasis in farm animals, pets and hobby animals.

6. The animal feed additive for use according to one of claims 1 to 5, **characterized in that** the active ingredient selected from diurnoside, cestrumoside and mixtures thereof is present in combination with a vitamin D3 metabolite, preferably selected from calcifediol and calcitriol.

7. The animal feed additive for use according to claim 6, **characterized in that** the ratio between the active ingredient selected from diurnoside, cestrumoside and mixtures thereof and the vitamin D3 metabolite preferably selected from calcifediol and calcitriol is in the range of 1:1,000 to 1,000:1, preferably in the range of 1:100 to 100:1.

8. The animal feed additive for use according to claim 1, **characterized in that** the active ingredient selected from diurnoside, cestrumoside and mixtures thereof is present in a dosage of 0.1 mg to 20,000 mg per kg of animal feed additive.

9. The animal feed for use for the prophylaxis of dyschondroplasia, osteochondrosis and/or leg weakness or for the prophylaxis of urolithiasis in farm animals, pets and hobby animals, **characterized in that** it contains an animal feed additive for use according to of claims 1 to 8 together with at least one feed component selected from the group consisting of protein carriers, carbohydrate carriers, roughage, silages, fats, vitamins, minerals and trace elements.

10. The animal feed for use according to claim 9, **characterized in that** it contains the active ingredient selected from diurnoside, cestrumoside and mixtures thereof in a dosage of 0.0001 mg to 3 mg per kilogram of feed, based on feed with 88 % dry matter.

11. A premix for preparing an animal feed for use according to claim 9 or 10, **characterized in that** it contains an animal feed additive for use according to one of claims 1 to 8 together with at least one feed component selected from the group consisting of protein carriers, carbohydrate carriers, roughage, silages, fats, vitamins, minerals and trace elements.

12. The animal feed additive for use according to one of claims 1 to 8, **characterized in that** the active ingredient is administered to the animal in an amount of 0.00001 mg to 0.3 mg per kilogram of body weight per day.

## Revendications

1. Additif alimentaire pour animaux pour l'utilisation dans un procédé d'inhibition de la protéine kinase C pour la prophylaxie de la dyschondroplasie, de l'ostéochondrose et/ou de la faiblesse des jattes ou pour la prophylaxie de la lithiase urinaire chez les animaux de ferme, les animaux de compagnie et les animaux de loisirs, **caractérisé en ce que** l'additif alimentaire pour animaux comprend du diurnoside, du cestrumoside ou un mélange de ceux-ci en combinaison avec un ou plusieurs autres ingrédients choisis parmi le magnolol, le honokiol et leurs mélanges, le mycélium d'Aspergillus niger, le mycélium d'Aspergillus oryzae et la chelérythrine.

2. Additif alimentaire pour animaux pour l'utilisation selon la revendication 1, **caractérisé en ce qu'**il contient du diurnoside, du cestrumoside ou un mélange de ceux-ci en combinaison avec du magnolol et/ou de l'honokiol.

3. Additif alimentaire pour animaux pour l'utilisation selon la revendication 1, **caractérisé en ce qu'**il contient du diurnoside, cestrumoside ou un mélange de ceux-ci en combinaison avec du mycélium d'Aspergillus niger ou d'Aspergillus oryzae.

4. Additif alimentaire pour animaux pour l'utilisation selon la revendication 1, **caractérisé en ce qu'**il contient du diurnoside, du cestrumoside ou un mélange de ceux-ci en combinaison avec de la chelérythrine.

5. Additif alimentaire pour animaux pour l'utilisation selon l'une des revendications 1 à 4 pour l'utilisation pour la prophylaxie de la dyschondroplasie, de l'ostéochondrose et/ou de la faiblesse des jattes ou pour la prophylaxie de la lithiase urinaire chez les animaux de ferme, les animaux de compagnie et les animaux de loisirs.

6. Additif alimentaire pour animaux pour l'utilisation selon l'une des revendications 1 à 5, **caractérisé en ce que** la substance active choisie parmi le diurnoside, le cestrumoside et les mélanges de ceux-ci est présente en combinaison avec un métabolite de la vitamine D3, de préférence choisi parmi le calcifédiol et le calcitriol.

7. Additif alimentaire pour animaux pour l'utilisation selon la revendication 6, **caractérisé en ce que** le rapport entre la substance active choisie parmi le diurnoside, le cestrumoside et les mélanges de ceux-ci et le métabolite de la vitamine D3 choisi de préférence parmi le calcifédiol et le calcitriol est compris entre 1:1000 et 1000:1, de préférence entre 1:100 et 100:1.

8. Additif alimentaire pour animaux pour l'utilisation selon la revendication 1, **caractérisé en ce que** la substance active choisie parmi le diurnoside, le cestrumoside et les mélanges de ceux-ci est présente à une dose de 0,1 mg à 20 000 mg par kg d'additif alimentaire pour animaux.

9. Additif alimentaire pour animaux pour l'utilisation pour la prophylaxie de la dyschondroplasie, de l'ostéochondrose et/ou de la faiblesse des jattes ou pour la prophylaxie de la lithiase urinaire chez les animaux de ferme, les animaux de compagnie et les animaux de loisirs, **caractérisé en ce qu'**il contient un additif alimentaire pour animaux pour l'utilisation selon l'une des revendications 1 à 8, conjointement avec au moins un composant d'aliment choisi dans le groupe constitué par les supports protéiques, les supports glucidiques, le fourrage grossier, les ensilages, les graisses, les vitamines, les minéraux et les oligo-éléments.

10. Additif alimentaire pour animaux pour l'utilisation selon la revendication 9, **caractérisé en ce qu'**il contient la substance active choisi parmi le diurnoside, le cestrumoside et les mélanges de ceux-ci à une dose de 0,0001 mg à 3 mg par kilogramme d'aliment, sur la base d'un aliment avec 88 % de matière sèche.

11. Prémélange pour préparer un aliment pour animaux pour l'utilisation selon la revendication 9 ou 10, **caractérisé en ce qu'**il contient un additif alimentaire pour animaux pour l'utilisation selon l'une des revendications 1 à 8, conjointement avec au moins un composant d'aliment choisi dans le groupe constitué par les supports protéiques, les supports glucidiques, le fourrage grossier, les ensilages, les graisses, les vitamines, les minéraux et les oligo-éléments.

12. Additif alimentaire pour animaux pour l'utilisation selon l'une des revendications 1 à 8, **caractérisé en ce que** la substance active est administrée à l'animal en une quantité de 0,00001 mg à 0,3 mg par kilogramme de poids corporel par jour.
